# EUROPEAN PATENT APPLICATION

(11) **EP 0 650 975 A1**
(43) Date of publication of application: **03.05.1995**
(21) Application number: 94112060.2
(22) Date of filing: 02.08.1994
(51) Int. Cl.: C07K 1/113

(54) **Refolding of denatured proteins using chaperonin 60 monomers from Thermus thermophilus**

(30) Priority: 03.08.1993 JP 210904/93
(71) Applicant: Nippon Oil Co., Ltd., Tokyo 105 (JP)
(72) Inventor: Yoshida, Masasuke, c/o Res. Lab. of Resources Util, Midori-ku, Yokohama-shi, Kanagawa (JP); Taguchi, Hideki, c/o Res. Lab. of Resources Util., Midori-ku, Yokohama-shi, Kanagawa (JP); Konishi, Jin, c/o Central Tech. Res. Laboratory, Naka-ku, Yokohama-shi, Kanagawa (JP)
(74) Representative: Boeters, Hans Dietrich, Dr.

(57) **Abstract**

A method for regeneration of denatured proteins by refolding of said denatured proteins by reacting said denatured proteins and one number selected from the group composed of ① chaperonin 60 monomer, ② chaperonin 60 monomer fragment which starts from threonine at 79th position from N-terminal of said chaperon 60 monomer and having a molecular weight of approximately 50 kD, ③ chaperonin 60 monomer and chaperonin 10 and ④ holo-chaperonin.

The refolding can be carried out without the addition of a high energy compound such as ATP and by merely adding one of the above mentioned proteins ① through ④ in the reaction system.

Inactivated proteins forming inclusion bodies in transformants or chemically inactivated proteins can be regenerated.

## Description

### Field of the Invention

This invention relates to processes for the refolding of denatured proteins using chaperonin 60 monomer and related substances thereof to regenerate the denatured proteins.

The present invention concerns chaperonin 60 monomer and related substances thereof which provide regenerating agents for denatured proteins by supporting and refolding them.

This invention can be applied for the regeneration and reactivation of chemically denatured inactive proteins and inactive proteins cumulated in transformants used in gene technology.

### Background of the Invention

Numerous types of proteins have been expressed and produced by gene technology procedures using host cells (transformants) such as Escherichia coli, yeasts or mammal derived cells.

For example, E. coli can be transformed with a gene coding for a protein having human derived physiological activity. Then, the transformed E. coli is cultured to express the target protein in the cultured cells. However, a large amount of cumulated protein in the cells becomes inactive by the formation of inclusion bodies, thus refolding of the inactive protein is required to give the target active protein. Therefore, up to now the expressed proteins have been denatured with a potent denaturing agent, such as guanidine hydrochloride, followed by refolding by dilution with a large amount of a diluent to reduce the concentration of the denaturing agent. However, the method resulted in a poor refolding rate and was liable to give differently bound thiol (-SH) groups. Thus, the method cannot always give the identical structure to that of the target active protein and is laborious. Therefore, production of the desired protein using E. coli has been limited to a few proteins such as growth hormones although the process is cost effective.

A new protein called a "molecular chaperon" which participates in the refolding of inactive protein was recently found. A molecular chaperon is defined as "a protein molecule which helps correct folding and aggregation of polypeptides but is not included in the final component of protein" by Ellis, J., (Nature, 328, 378-379 (1987)). Taguchi et al. (Biophysics, 33(1) 11-14 (1993)) stated that a molecular chaperon is required for steps wherein the conformational coupling of protein varies greatly such as the synthesis of proteins, folding of newly synthesized polypeptide chains, permeation of various membranes, heat shocks and degradation of proteins at the alteration of generation. In other words, a molecular chaperon is indispensable throughout the lifespan of proteins.

Chaperonin is a typical chaperon and has been found to possess an extensive homogeneity with the primary structures of GroEL which is required for the formation of bacteriophages in E. coli and a binding protein of Rubisco, ribulose-1,5-bisphosphate carboxylase, to help the aggregation of Rubisco in chlorophyll. Thus both GroEL and Rubisco and their related proteins were proposed to be collectively named chaperonin.

Chaperonin is constructed from two subunits, viz. chaperonin 60 having a molecular weight of approximately 60 kD, and chaperonin 10 having an adjunctive role. An electron microscopic observation revealed that the structure of chaperonin could be interpreted as a particle having two overlaying rings each composed of seven molecules of chaperonin 60 and, furthermore, an overlaying ring composed of seven molecules of chaperonin 10.

Chaperonin 60 monomer is a constitutional element of chaperonin 60 and exhibits a molecular weight of approximately 58 kD with SDS-polyacrylamide gel electrophoresis (SDS-PAGE). The molecular weight of chaperonin 10 monomer is approximately 10 kD with SDS-PAGE.

GroEL, a chaperonin 60, derived from E. coli and GroES corresponding to chaperonin 10 can be purified separately. However, GroEL and GroES in a chaperonin derived from thermophilic bacteria Thermus thermophilus bind firmly and are known to be purified as a complex of chaperonin 60 and chaperonin 10 (J. Biol. Chem., 266, 22411-22418 (1991)).

The inventors have been investigating the active site of the above mentioned chaperonin and found that chaperonin 60 monomer obtained from Thermus thermophilus inhibits the aggregation of un-natural substrate proteins without the addition of ATP or any troublesome procedure, and accelerates the refolding of proteins, and accomplished the present invention.

Furthermore, the inventors found similar activities for a fragment of chaperonin 60 monomer, which starts from threonine at 79th position from the N-terminal and having molecular weight of approximately 50 kD, for chaperonin 60 monomer and chaperonin 10, and for holo-chaperonin.

### Summary of the Invention

One object of the present invention is to provide a method for the regeneration of denatured proteins by refolding the denatured proteins, particularly inactive proteins expressed and cumulated in transformed microorganisms, by a simple procedure without using a specific reagent such as ATP.

Another object of the present invention is to provide a regenerating agent applicable for the regeneration of the denatured protein.

Therefore, the present invention relates to a method for regeneration of denatured proteins by refolding them with one of the following agents composed of ① chaperonin 60 monomer, ② chaperonin 60 monomer fragment which starts from threonine at 79th position from N-terminal of said chaperonin 60 monomer and having a molecular weight of approximately 50 kD, ③ chaperonin 60 monomer and chaperonin 10, and ④ holo-chaperonin.

In addition, the present invention relates to regeneration agents for denatured proteins composed of one of the above mentioned ①, ②, ③ and ④ proteins.

The amino acid sequence from the N-terminal of chaperonin 60 monomer was determined by hydrolysis with an immobilized trypsin followed by a reverse HPLC. The molecular weight was estimated with polyacrylamide gel electrophoresis (PAGE).

### Brief Description of the Drawing

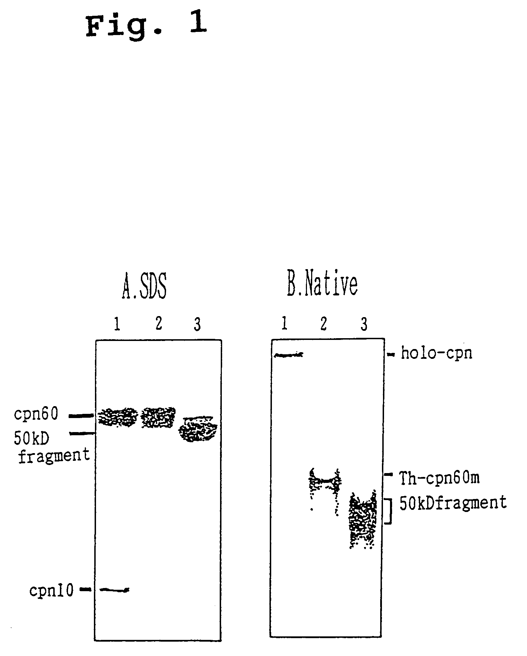

Fig. 1 shows the results of PAGE of chaperonin 60 monomer, its fragment of 50 kD and chaperonin 10 obtained by the Example 1 in the presence (A) and absence (B) of SDS.

### Detailed Description of the Invention and Preferred Embodiment

This invention is accomplished on the bases of the above mentioned findings. Denatured proteins according to the present invention comprise those inactive proteins which are expressed and cumulated in large amounts in transformants of E. coli and the like prepared by gene technology or treated with a protein regenerating agent.

Chaperonin 60 monomer can be obtained from purified protein derived from Thermus thermophilus. That is, culturing of Thermus thermophilus, degradation of the harvested cells, separation of the supernatant and treatment with 30-60% saturated ammonium sulfate. The precipitated fraction is dissolved in a buffer, dialyzed, subjected to a DE-cellulose column and eluted with 250 mM NaCl solution. The eluate is ultrafiltered, evaporated, subjected to a Sepharose CL6B column to collect the void volume fractions and purified to give holo-chaperonin comprising chaperonin 60 monomer and chaperonin 10.

The purified holo-chaperonin is denatured in 0.1% trifluoroacetic acid, subjected to a reverse HPLC and eluted with acetonitrile to collect peak fractions containing chaperonin 60 monomer and chaperonin 10, separately.

A 50 kD fragment of the chaperonin 60 monomer can be obtained by digesting the above mentioned chaperonin 60 monomer with an immobilized trypsin and purification with gel permeation HPLC.

The reaction of the denatured protein and one of the above mentioned ①-④ proteins may be carried out directly in a buffer at pH 7-8. Preferably, one of the above mentioned ①-④ proteins is immobilized on a suitable carrier such as TSK gel (Tosoh) or AF-tresil Toyopearl 650M, then the solution of the above mentioned denatured protein is passed through the column packed with the above mentioned protein to refold and regenerate the denatured protein.

According to the present invention, the denatured protein can be refolded by merely adding the above mentioned chaperonin 60 monomer and the like to a system containing the denatured protein. Furthermore, the procedure of the present invention can be carried out without addition of a high energy compound such as ATP, although conventional refolding and regeneration procedures require these high energy compounds. Immobilization of chaperonin 60 monomer on a resin results in a simple and easily operable reaction.

The present invention has further characteristic features viz. the chaperonin of the present invention is stable against heat and prevents the denaturation of the substrate protein during the procedure of refolding.

The present invention will be explained by the following examples. However the scope of the present invention is not restricted by these examples.

### Example 1

### Preparation of chaperonin 60 monomer and its fragment:

Holo-chaperonin was prepared from Thermus thermophilus HB8 (ATCC 27634) and purified according to a known method [J. Biol. Chem., 266, 22411-22418 (1991)].

The resultant purified holo-chaperonin was denatured by treatment with 0.1% trichloroacetic acid, subjected to reverse HPLC (Sensyu Pak C4 Hi-Pore Co (uer)), eluted with acetonitrile in a linear gradation. The eluted peak fractions containing chaperonin 60 monomer were dried in vacuo, dissolved in a buffer containing 6M guanidine HCl, diluted to 40-fold with 50 mM Tris-HCl buffer, pH 7.5, at 60°C to give chaperonin 60 monomer. Chaperonin 10 was also isolated in the same treatment.

Fifteen milligrams of the chaperonin 60 monomer was dissolved in three milliliter of 50 mM Tris-HCl buffer, pH 7.5, and treated with 2.8 unit of immobilized TPCK-trypsin (Picrce) for three hrs. at 25°C.

The treated chaperonin 60 monomer was subjected to PAGE in the presence (A) or absence (B) of SDS (sodium dodecylsulfate). The results are shown in Fig. 1. In Fig.1, numerals 1, 2 and 3 represent holo-chaperonin (holo-cpn), chaperonin 60 monomer (Th-cpn60m) and its 50 kD fragment, respectively. The concentration of PAGE was 13% in (A) and 7.5% in (B), respectively; seven micrograms of these proteins were used and the gel was stained with Coomasie brilliant blue.

### Example 2

Bovine mitochondrial rhodanese (Sigma) was denatured and inactivated in 50 mM phosphate buffer, pH 7.8, containing 6M guanidine HCl and 5 mM dithiothreitol. The resultant mixture was diluted at a ratio of 1:25 and at 37°C with 50 mM phosphate buffer containing 50 mM of dithiothreitol, 50 mM of sodium thiosulfate, 3.3 µM of chaperonin 60 monomer (Th-cpn60m) obtained by the preceding Example 1, a mixture of the monomer and chaperonin 10 (Th-cpn10), or a 50 kD fragment of chaperon 60 monomer (50 kD fragment), to give a final concentration of rhodanese at 0.13 µM. After 60 minutes, a portion of the reaction solution was drawn off and the rhodanese activity was determined. The results are shown in Table 1A. The rhodanese activities in Th-cpn 60m and Th-cpn10 with added Mg ATP are shown as references.

### Example 3

### Reaction of protein immobilized on resin

In a 0.1M phosphate buffer containing 0.5M NaCl, pH 7.5, six milligrams of a resin (AF-tresil Toyopearl 650) were added, then a Th-cpn 60m solution prepared in a similar manner to that of Example 2 was added and stirred at 4°C overnight. The resultant resin was washed with 0.5M NaCl and caused to react with 0.1M Tris-HCl buffer containing 0.5M NaCl, pH 8.0, for one hr. at 25°C to block an excess amount of active groups to immobilize 20 µg of the protein. The obtained resin was stirred in the diluted buffer solution of Example 2 to cause a refolding reaction. The results are shown in Table 1B. A similar treatment was carried out using immobilized bovine serum albumin as a control. The regeneration rate of the sample with added chaperonin was calculated as specific activity compared to that of before denaturation, which was taken as 100%.

**Table 1**

| | Additive | Regeneration rate (%) |
|---|---|---|
| A. | None | 4.5 |
| | Th-cpn60m | 24.7 |
| | Th-cpn60m + Th-cpn10 | 24.6 |
| | Th-cpn60m + Th-cpn10 + Mg ATP | 25.2 |
| | 50 kD fragment | 35.0 |
| B. | immobilized BSA | 0.0 |
| | immobilized Th-cpn60m | 10.0 |

## Claims

1. A method for regeneration of denatured proteins characterized by refolding said denatured proteins by reacting said denatured proteins and one protein selected from the group composed of ① chaperonin 60 monomer, ② chaperonin 60 monomer fragment which starts from threonine at 79th position from N-terminal of said chaperon 60 monomer and having a molecular weight of approximately 50 kD, ③ chaperonin 60 monomer and chaperonin 10, and ④ holo-chaperonin.

2. A method according to claim 1 wherein said proteins ① through ④ are derived from Thermus thermophilus and purified.

3. A method according to claim 1 wherein said denatured proteins are inactivated proteins cumulated in transformed microorganisms.

4. A method for regeneration of denatured proteins characterized by immobilizing one of the proteins ① through ④ according to claim 1 on a resin and contacting with said denatured proteins.

5. A denatured protein regenerating agent composed of chaperonin 60 monomer.

6. A denatured protein regenerating agent composed of a chaperonin 60 monomer fragment starting from threonine at 79th position from N-terminal of said chaperonin 60 monomer and having a molecular weight of approximately 50 kD.

7. A denatured protein regenerating agent composed of chaperonin 60 monomer and chaperonin 10.

8. A denatured protein regenerating agent composed of holo-chaperonin.
